# EUROPEAN PATENT SPECIFICATION

(11) **EP 3 067 691 B1**
(45) Date of publication and mention of the grant of the patent: **31.03.2021**
(21) Application number: 14860586.8
(22) Date of filing: 31.10.2014
(51) Int. Cl.: G01N 29/12, G01N 3/30, G01N 33/08, G01N 29/04, G01N 29/46

(54) **EXAMINATION DEVICE**
UNTERSUCHUNGSVORRICHTUNG
DISPOSITIF D'EXAMEN

(30) Priority: 06.11.2013 JP 2013230106
(43) Date of publication of application: 14.09.2016
(73) Proprietor: Kyowa Machinery Co., Ltd., Okayama 708-1115 (JP)
(72) Inventor: OTOMO, Masaki, Sapporo-shi, Hokkaido 006-0807 (JP)
(74) Representative: Hoffmann Eitle
(86) International application number: PCT/JP2014/079026
(87) International publication number: WO 2015/068644

(56) References cited:
- EP-A1- 0 738 888
- EP-A2- 0 295 755
- JP-A- S6 472 030
- JP-A- H01 174 935
- JP-A- H06 294 724
- JP-A- H10 227 766
- JP-A- 2003 057 216
- US-A- 3 744 299

## Description

### TECHNICAL FIELD

The present invention relates to examination devices including hammer portions for tapping objects, and detection portions for detecting vibrations caused by the hammer portions tapping the objects.

### BACKGROUND ART

Conventionally, there have been known examination devices including hammer portions for tapping objects (for example, hen's eggs), and detection portions for detecting vibrations caused by the hammer portions tapping the objects (refer to Patent Document 1, for example). These examination devices are configured to examine objects, based on sound signals detected by the detection portions.

On the other hand, these examination devices are configured to convert sound signals detected by the detection portion into spectral intensities in frequency bands, in order to examine objects. However, these examination devices have induced degradation of the detection accuracy, due to variations of the sizes of objects, changes of the examination environments, and the like.

### PRIOR ART DOCUMENTS

### PATENT DOCUMENTS

Patent Document 1: JP-A-10-227766

EP 0 295 755 A2 relates to a method for testing eggs for the existence of cracks or holes in the egg shells by examining the elasticity of said shells and an apparatus for carrying out said process.

EP 0 738 888 A1 relates to a probe, device and method for testing eggs.

US 3 744 299 A relates to a crack detector.

### SUMMARY OF THE INVENTION

### PROBLEMS TO BE SOLVED BY THE INVENTION

Accordingly, in view of the aforementioned circumstances, it is an object of the present invention to provide an examination device capable of having improved detection accuracy.

### MEANS FOR SOLVING THE PROBLEMS

According to the present invention, there is provided an examination device as claimed in claim 1.

With the examination device according to the present embodiment, the detection portion detects the vibration caused by the hammer portion tapping the object. In this case, if the object is a normal hen's egg, the hammer portion bounds on the object. On the other hand, if the object is an abnormal hen's egg (such as a hen's egg having a crack), the object absorbs the impact of the hammer portion, which inhibits the hammer portion from bounding on the object.

Further, the calculation portion calculates the number of times the hammer portion has bounded to impinge on the object regarding a single tapping operation of the hammer portion, based on the vibration detected by the detection portion. The determination portion determines the quality of the object, based on the number of times calculated by the calculation portion. This can improve the detection accuracy.

According to the present invention, the detection portion is a piezoelectric device mounted to a tip end portion of the hammer portion.

With this structure, the detection portion is a piezoelectric device mounted to the tip end portion of the hammer portion, which can suppress the vibrations detected by the detection portion from containing noises, such as environmental noises, for example. This can effectively improve the detection accuracy.

Also, the examination device according to the present invention may have a configuration in which:
the calculation portion includes a first impingement calculation portion configured to calculate time of a first impingement, and a second impingement calculation portion configured to determine, through a calculation, presence or absence of a second impingement,
the first impingement calculation portion calculates a largest vibration strength, as a first impingement, out of the vibration detected by the detection portion,
the second impingement calculation portion detects a largest vibration strength within a set time period after the first impingement, and the second impingement calculation portion determines that there has been a second impingement when this vibration strength is larger than a set value and determines that there has been no second impingement when this vibration strength is smaller than the set value, and
the determination portion is configured to determine that the object is not good, when the second impingement calculation portion determines that there has been no second impingement.

Also, the examination device according to the present invention may have a configuration in which:
the calculation portion further includes a third impingement calculation portion configured to determine, through a calculation, presence or absence of a third impingement,
the third impingement calculation portion detects a largest vibration strength within a set time period after the second impingement when the second impingement calculation portion determines that there has been the second impingement, and the third impingement calculation portion determines that there has been a third impingement when this vibration strength is larger than a set value and determines that there has been no third impingement when this vibration strength is smaller than the set value, and
the determination portion determines that the object is not good, when the third impingement calculation portion determines that there has been no third impingement.

Also, the examination device according to the present invention may have a configuration in which:
the calculation portion calculates a number of times the vibration strength of the vibration detected by the detection portion has exceeded a set value, as a number of impingements, and
the determination portion determines that the object is good when the number of impingements calculated by the calculation portion is equal to or more than a set number of times, and determines that the object is not good, when the number of impingements calculated by the calculation portion is less than the set number of times.

### EFFECT OF THE INVENTION

As described above, the examination device according to the present invention has an excellent advantage of having improved detection accuracy.

### BRIEF DESCRIPTION OF THE DRAWINGS

Fig. 1 illustrates a schematic general view of an examination device according to an embodiment of the present invention.
Fig. 2 illustrates a plan view of an entire percussion unit according to the same embodiment.
Fig. 3 illustrates a schematic general view of a percussion portion according to the same embodiment.
Fig. 4 illustrates a schematic general view illustrating an operation of the percussion portion according to the same embodiment.
Fig. 5 illustrates a schematic general view illustrating an operation of the percussion portion according to the same embodiment.
Fig. 6 illustrates a flow chart of an examination according to the same embodiment.
Fig. 7 illustrates a time-vibration strength graph illustrating an examination according to the same embodiment, and illustrates a graph resulted from examination of a normal product.
Fig. 8 illustrates a time-vibration strength graph illustrating an examination according to the same embodiment, and illustrates a graph resulted from examination of an abnormal product.
Fig. 9 illustrates a time-vibration strength graph illustrating an examination according to another embodiment of the present invention, and illustrates a graph resulted from examination of a normal product.
Fig. 10 illustrates a time-vibration strength graph illustrating an examination according to the same embodiment, illustrating a graph resulted from examination of an abnormal product.

### MODE FOR CARRYING OUT THE INVENTION

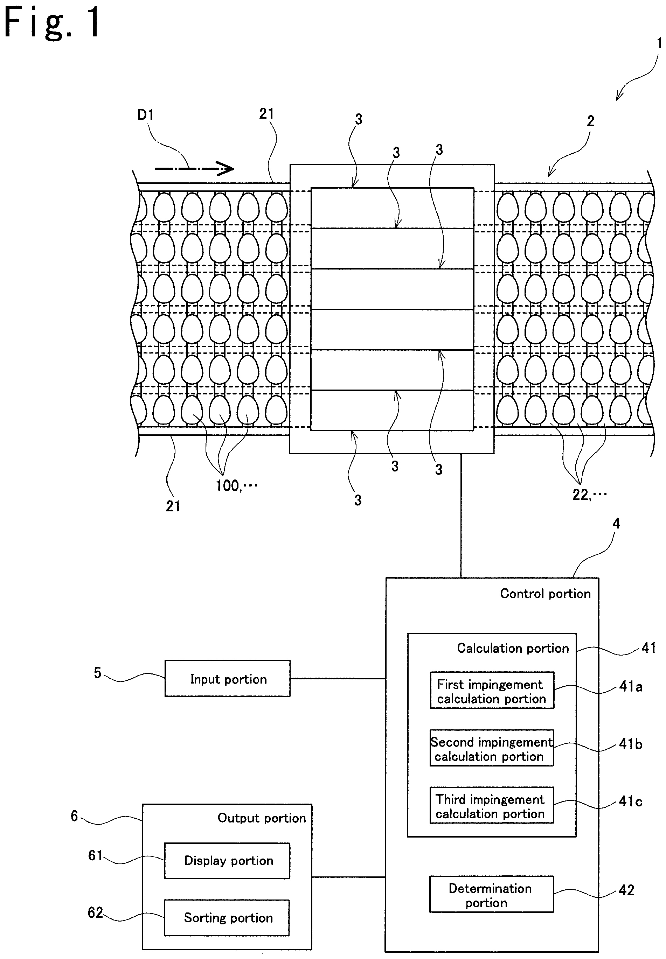

Hereinafter, with reference to Figs. 1 to 8, an embodiment of an examination device according to the present invention will be described. The examination device according to the present embodiment is an egg crack examination device for examining the presence or absence of cracks (breakages) in eggs, assuming that eggs (particularly, hen's eggs) are objects to be examined. Further, in the respective drawings, dimension ratios in the drawings are not necessarily equal to actual dimension ratios.

As illustrated in Fig. 1, the examination device 1 according to the present embodiment includes a transfer device 2 for transferring eggs 100 in the transferring direction D1, a plurality of percussion units 3 which are placed above the transfer device 2 and are configured to percuss the eggs 100 being transferred by the transfer device 2, and a control portion 4 for controlling the device. Further, the examination device 1 includes an input portion 5 for inputting information about examinations thereto, and an output portion 6 for outputting information about examinations and the like.

The transfer device 2 includes a pair of travelling members (for example, chains or belts) configured to travel in such a way as to rotate in an endless manner, and a plurality of rotating members 22, ··· which are placed along the horizontal direction orthogonal to the transferring direction D1 and are rotatably mounted to the pair of the travelling members 21 and 21 at even intervals. Thus, the transfer device 2 transfers the eggs 100 being placed to straddle the rotating members 22, along the transferring direction D1, while rotating the eggs 100.

The rotating members 22 include recess-shaped holding portions 22a for holding the eggs 100. A plurality of the holding portion 22a are arranged along the longitudinal direction of the rotating members 22. Thus, the transfer device 2 transfers the eggs 100 while aligning the eggs 100 in the transferring direction D1 and, further, arranges a plurality of these rows of eggs being transferred (six rows in the present embodiment) in the longitudinal direction of the rotating members 22.

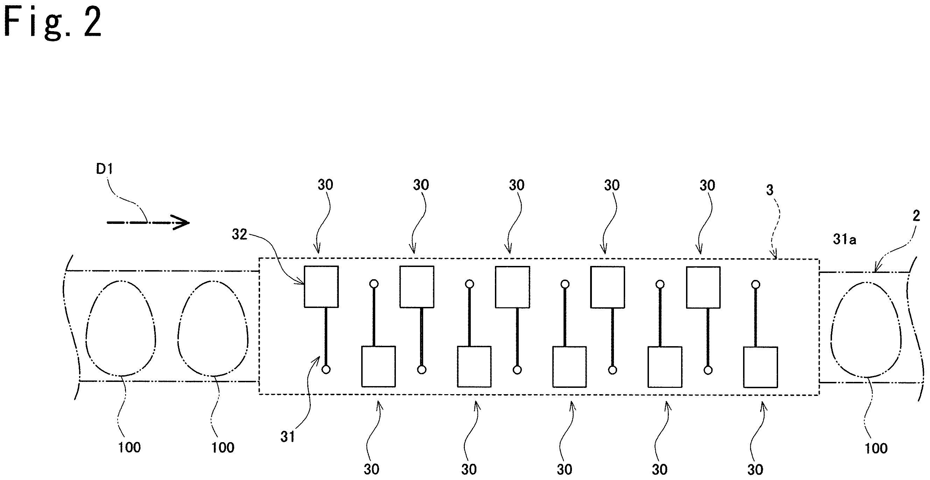

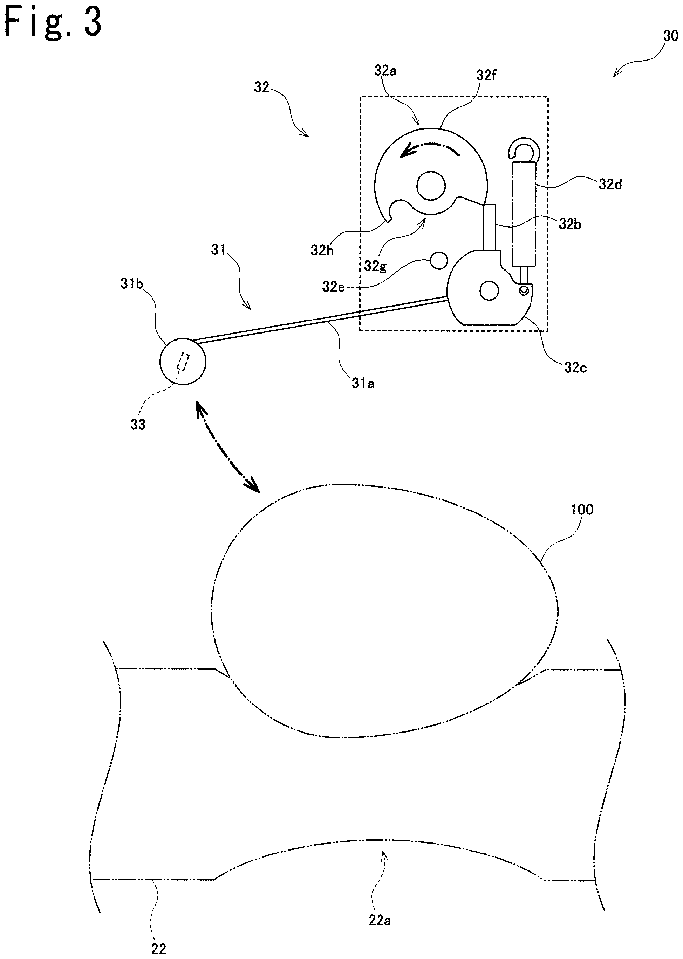

The percussion units 3 are placed for the respective rows of the eggs 100 being transferred. In the present embodiment, six percussion units 3 are provided. More specifically, the plurality of the percussion units 3 are arranged in the direction orthogonal to the transferring direction D1. Further, as illustrated in Figs. 2 and 3, the percussion units 3 include a plurality of percussion portions 30, ··· for tapping the eggs 100.

The percussion portions 30 include a hammer portion 31 for tapping an egg 100, a driving portion 32 for operating the hammer portion 31, and a detection portion 33 for detecting vibrations caused by the hammer portion 31 tapping the egg 100. Further, the plurality of the percussion portions 30 are arranged along the transferring direction D1. In the present embodiment, ten percussion portions 30 are provided in a single percussion unit 3.

The hammer portion 31 includes an elongated member 31a formed to have an elongated length, and a touching portion 31b which is coupled to the tip end portion of the elongated member 31a and is configured to touch on the egg 100. Further, the hammer portion 31 is structured such that, if the hammer portion 31 taps a normal egg 100, the elongated member 31a warps, so that the touching portion 31b bounds on the surface of the egg 100 to impinge thereon multiple times.

More specifically, the elongated member 31a is formed to have a round bar shape and has elasticity such that the elongated member 31a warps when the hammer portion 31 taps an egg 100. For example, the elongated member 31a is formed from a carbon fiber bar, or a metal bar made of an Ni-Ti alloy or the like. Further, for example, the touching portion 32b is made of a resin such as polyacetal, or the like.

The driving portion 32 includes a rotatable cam 32a, a rod-shaped contact body 32b which comes into contact with the cam 32a at its tip end portion, and a revolving portion 32c which is coupled to the base end portion of the elongated member 31a and the base end portion of the contact body 32b and causes the hammer portion 31 and the contact body 32b to revolve about an axis. Further, the driving portion 32 includes a biasing member 32d such as a spring for biasing the touching portion 31b of the hammer portion 31 toward an egg 100, and a restriction portion 32e configured to restrict the hammer portion 31 and the contact body 32b from revolving by a predetermined angle or more since the contact body 32b touches therewith.

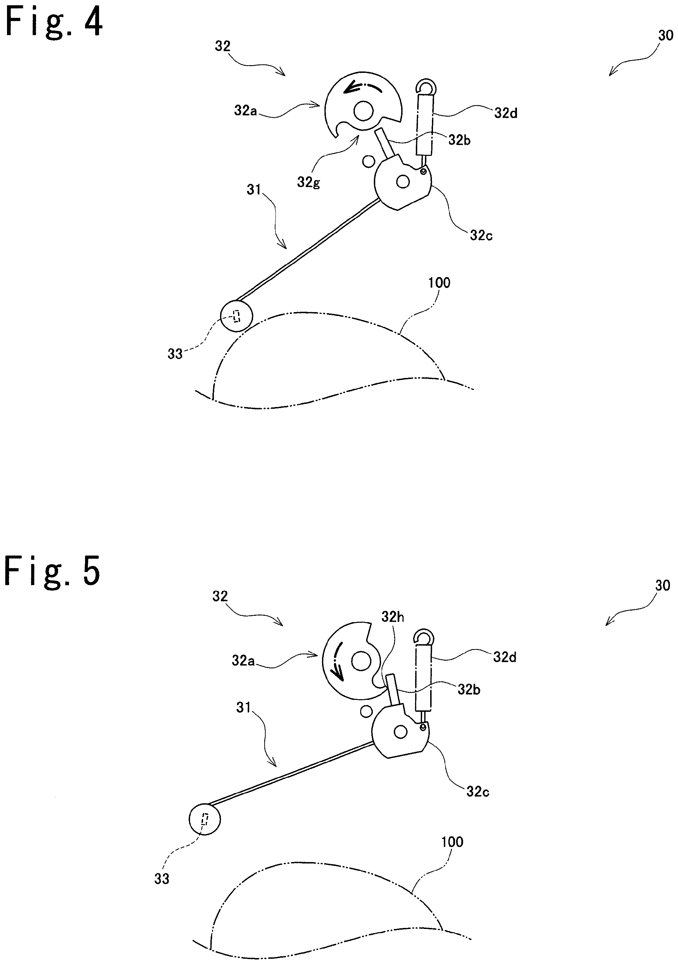

The cam 32a is provided, at its outer peripheral portion, with a sliding contact portion 32f which comes into sliding contact with the contact body 32b, in order to hold the hammer portion 31 at a standby position (see Fig. 3) at which the hammer portion 31 is on standby in such a way as to be spaced apart from the egg 100. Further, the cam 32a includes a recessed portion 32g for releasing the contact of the cam 32a with the contact body 32b for causing the hammer portion 31 to tap the egg 100 (see Fig. 4), and a locking piece 32h for locking the contact body 32b, in order to restore the hammer portion 31 to the standby position (see Fig. 5).

The detection portion 33 is a piezo-electric device configured to convert vibrations exerted thereon from the outside into voltages through a piezo-electric effect, for electrically detecting the vibrations. Further, the detection portion 33 is mounted to the tip end portion of the hammer portion 31. More specifically, the detection portion 33 is placed inside the touching portion 31b.

The control portion 4 includes a calculation portion 41 configured to calculate the number of times each hammer portion 31 has impinged on an egg 100 regarding a single tapping operation of the hammer portion 31, based on the vibration detected by the detection portion 33. Further, the calculation portion 41 includes a determination portion 42 configured to determine the quality of the egg 100, based on the number of times calculated by the calculation portion 41.

The calculation portion 41 includes a first impingement calculation portion 41a configured to calculate the time when each hammer portion 31 impinged on an egg 100 at first. Further, the calculation portion 41 includes a second impingement calculation portion 41b configured to determine, through a calculation, the presence or absence of a second impingement due to bounces of each hammer portion 31 on the surface of the egg 100, and a third impingement calculation portion 41c configured to determine, through a calculation, the presence or absence of a third impingement.

The output portion 6 includes a display portion 61 configured to display, on a screen, information about vibrations detected by the detection portions 33, results of determinations performed by the determination portion 42, and the like, and a sorting portion 62 configured to sort eggs 100 based on the results of determinations by the determination portion 42. The sorting portion 62 sorts out eggs 100 determined to be normal products from eggs 100 determined to be abnormal products and, further, transfers them to respective different downstream devices.

The examination device 1 according to the present embodiment has the structure described above. Next, with reference to Figs. 3 to 5, there will be described operations of the percussion portions 30 according to the present embodiment.

As illustrated in Fig. 3, when the sliding contact portion 32f of the cam 32a is in sliding contact with the contact body 32b, the hammer portion 31 is held at the standby position at which the hammer portion 31 is spaced apart from the egg 100. At this time, the spring as the biasing member 32d is in a state of being stretched, which biases the contact body 32b toward the cam 32a.

Further, as illustrated in Fig. 4, if the cam 32a is rotated, the rotation of the cam 32a releases the contact between the cam 32a and the contact body 32b due to the recessed portion 32g. Thus, the hammer portion 31, the contact body 32b and the revolving portion 32c which are being biased toward the biasing member 32d are revolved integrally, which causes the hammer portion 31 to tap the egg 100. Further, even when the hammer portion 31 is being in contact with the egg 100, the biasing member 32d is in a state of being stretched, which continuously biases the hammer portion 31 toward the egg 100.

In this case, if the hammer portion 31 taps a normal egg 100, the hammer portion 31 bounds on the surface of the egg 100 and, thus, repeatedly impinges on the egg 100 multiple times. On the other hand, if the hammer portion 31 taps an abnormal egg 100 having a crack, the egg 100 absorbs the impact of the hammer portion 31. This inhibits the hammer portion 31 from bounding on the surface of the egg 100, which causes the hammer portion 31 to impinge on the egg 100 a smaller number of times than that in cases of normal eggs 100.

Further, if the cam 32a is further rotated, the locking piece 32h locks the contact body 32b, as illustrated in Fig. 5. Thus, along with the rotation of the cam 32a, the hammer portion 31 is revolved in such a way as to be separated from the egg 100. Thereafter, as illustrated in Fig. 3, the hammer portion 31 is restored to the standby position. As described above, a single tapping operation of the hammer portion 31 refers to an operation for rotating the cam 32a once for causing the hammer portion 31 to move from the standby position to tap an egg 100 and for restoring the hammer portion 31 to the standby position again.

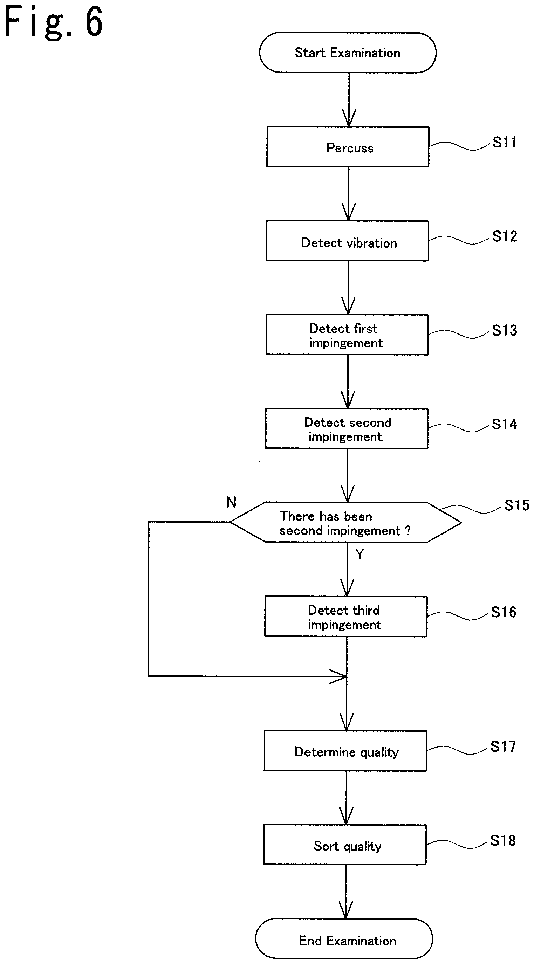

Next, with reference to Figs. 6 to 8, there will be described an examination method with the examination device 1 according to the present embodiment.

Each hammer portion 31 taps an egg 100 being transferred by the transfer device 2 (step 11). Then, the detection portion 33 detects the vibration caused by the hammer portion 31 tapping the egg 100 (step 12). Thereafter, the first impingement calculation portion 41a calculates a largest vibration strength S1, as a first impingement, out of information about the vibration detected by the detection portion 33 (step 13).

Further, the second impingement calculation portion 41b detects a largest vibration strength S2 within a set time period Ti after the first impingement, and determines, through a calculation, whether or not this vibration strength S2 corresponds to a second impingement (step 14). More specifically, the second impingement calculation portion 41b determines that there has been a second impingement, if this vibration strength S2 is larger than a set value V1.

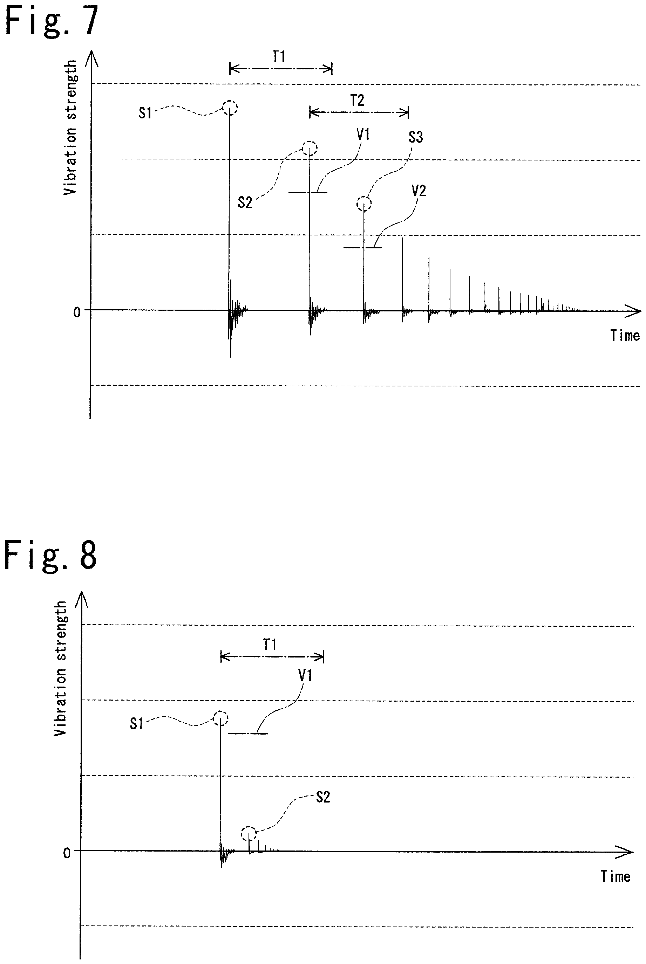

As illustrated in Fig. 7, if there has been a second impingement ("Y" in step 15), the third impingement calculation portion 41c detects a largest vibration strength S3 within a set time period T2 after the second impingement. Further, the third impingement calculation portion 41c determines, through a calculation, whether or not this vibration strength S3 corresponds to a third impingement (step 16). More specifically, the third impingement calculation portion 41c determines that there has been a third impingement, if this vibration strength S3 is larger than a set value V2. Further, as illustrated in Fig. 8, if there has been no second impingement ("N" in step 15), the third impingement calculation portion 41c performs no calculation.

Further, when the hammer portion 31 has impinged on the egg 100 three times as illustrated in Fig. 7, the determination portion 42 determines that the examined egg 100 is a normal product. When the hammer portion 31 has not impinged on the egg 100 three times as illustrated in Fig. 8, the determination portion 42 determines that the examined egg 100 is an abnormal product (step 17). Thereafter, based on the result of the determination by the determination portion 42, the sorting portion 62 sorts out normal products from abnormal products (step 18).

Further, the eggs 100 are percussed by all the percussion portions 30,··· (the ten percussion portions), and the respective percussion portions 30 tap the eggs 100 at slightly different positions. Further, the eggs 100 are rotated by the transfer device 2, so that the examination device 1 examines the eggs 100 by percussing them over the entire areas thereof, through the plurality of the percussion portions 30,···.

As described above, in the examination device 1 according to the present embodiment, each detection portion 33 detects the vibration caused by the hammer portion 31 tapping an egg 100. In this case, if the hammer portion 31 taps a normal egg 100, the hammer portion 31 bounds at the surface of the egg 100. But if the hammer portion 31 taps an abnormal egg 100, the egg 100 absorbs the impact of the hammer portion 31, which inhibits the hammer portion 31 from bounding on the surface of the egg 100.

Further, the calculation portion 41 calculates the number of times the hammer portion 31 has bounded to impinge on the egg 100 regarding a single tapping operation of the hammer portion 31, based on the vibration detected by the detection portion 33. Thereafter, the determination portion 42 determines the quality of the egg 100, based on the number of impinges calculated by the calculation portion 41. This can improve the detection accuracy.

Further, in the examination device 1 according to the present embodiment, each detection portion 33 is a piezoelectric device mounted to the tip end portion of the hammer portion 31. This can suppress vibrations detected by each detection portion 33 from containing noises (for example, environmental noises, vibrations caused by the other hammer portions 31 tapping eggs 100, and the like), in comparison with structures for detecting tapping sounds as in conventional examination devices. This can effectively improve the detection accuracy.

Further, the examination device according to the present invention is not limited to the structure according to the aforementioned embodiment and, further, is not limited to the aforementioned effects and advantages. For example, it is also possible to arbitrarily select structures, methods and the like in various modification examples as follows and to employ them as the structures, methods and the like according to the aforementioned embodiment, as a matter of course.

In the examination device 1 according to the aforementioned embodiment, the calculation portion 41 includes the second and third impingement calculation portions 41b and 41c, the second impingement calculation portion 41b is configured to determine, through a calculation, the presence or absence of a second impingement, and the third impingement calculation portion 41c is configured to determine, through a calculation, the presence or absence of a third impingement. However, the examination device according to the present invention is not limited to this structure.

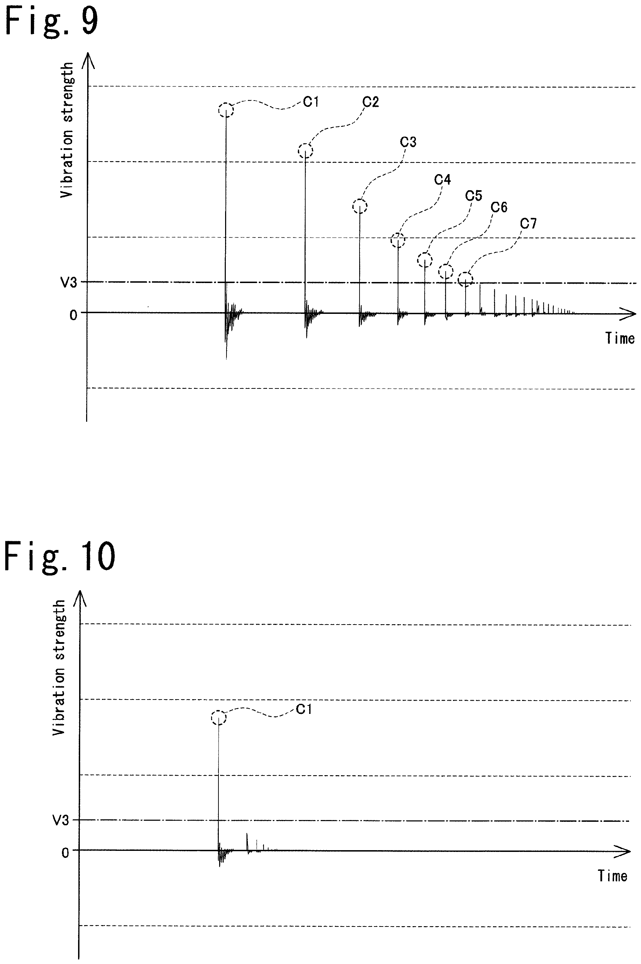

For example, in the examination device according to the present invention, the calculation portion 41 can be also 19 configured to detect the number of times each hammer portion 31 has impinged on an egg 100, as illustrated in Figs. 9 and 10. With this structure, as illustrated in Figs. 9 and 10, the calculation portion 41 calculates the number of times the vibration strength has exceeded a set vibration strength value V3, as the number of impingements C1, C2,···, Cn. Further, if the hammer portion 31 has impinged thereon a set number of times (for example, three times) or more, the determination portion 42 determines that the examined egg 100 is a normal product.

Further, in the examination device 1 according to the aforementioned embodiment, the determination portion 42 is configured to determine that an examined egg 100 is a normal product, if the hammer portion 31 has impinged on the egg 100 three times. However, the examination device according to the present invention is not limited to this structure. For example, in the examination device according to the present invention, the determination portion 42 can be also configured to determine that an examined egg 100 is a normal product, if the hammer portion 31 has impinged on the egg 100 two times or four times or more.

Further, in the examination device 1 according to the aforementioned embodiment, the detection portions 33 are piezoelectric devices mounted to the tip end portions of the hammer portions 31.

Further, in the examination device 1 according to the aforementioned embodiment, each hammer portion 31 is configured to tap an egg 100 by being biased toward the biasing member 32d such as a spring. However, the examination device 1 according to the present invention is not limited to this structure. For example, in the examination device according to the present invention, the driving portions 32 does not include the biasing member 32d, and the hammer portions 31 can be configured to tap eggs utilizing free fall.

Further, in the examination device 1 according to the aforementioned embodiment, the transfer device 2 is configured to transfer eggs 100 placed to straddle the rotating members 22, 22, along the transferring direction D1, while rotating the eggs 100. However, the examination device according to the present invention is not limited to this structure. For example, in the examination device according to the present invention, the transfer device 2 can be also configured to hold a plurality 21 of eggs 100 on a palette and, further, to transfer the eggs 100 together with the palette along the transferring direction D1.

Further, in the examination device 1 according to the aforementioned embodiment, the transfer device 2 is configured to arrange a plurality of rows of eggs being transferred, for aligning and transferring the eggs 100 along the transferring direction D1. However, the examination device according to the present invention is not limited to this structure. For example, in the examination device according to the present invention, the transfer device 2 can be also configured to include a single row of eggs being transferred, for aligning and transferring the eggs 100 along the transferring direction D1.

Further, the examination device 1 according to the aforementioned embodiment is configured to include the plurality of the percussion portions 30. However, the examination device according to the present invention is not limited to this structure. For example, the examination device according to the present invention can be also configured to include a single percussion portion 30.

Further, in the examination device 1 according to the aforementioned embodiment, the percussion portions 30 are configured to include the driving portion 32 utilizing the cam. However, the examination device according to the present invention is not limited to this structure. For example, in the examination device according to the present invention, the percussion portions 30 can be also configured to include a driving portion utilizing a solenoid, such that this driving portion causes the hammer portion 31 to revolve, through reciprocating operations of the solenoid.

Further, the examination device 1 according to the aforementioned embodiment is configured such that objects are eggs 100. However, the examination device according to the present invention is not limited to this structure. For example, the examination device according to the present invention can be also configured such that objects are table-tennis balls (ping-pong balls), packaging cans and the like.

Further, the examination device according to the present invention can be also configured such that the set time periods T1 and T2 and the set vibration strength values V1 and V2 for use in calculations by the second impingement calculation portion 41b and the third impingement calculation portion 41c are changed to information inputted through the input portion 5.

Further, in the present invention, the examination device 1 can be used to output results of determinations of qualities of eggs 100 to the display portion 61, and the eggs 100 can be sorted (abnormal products can be eliminated therefrom) through operators hands.

### DESCRIPTION OF REFERENCE SIGNS

- 1: Examination device
- 2: Transfer device
- 3: Percussion unit
- 4: Control portion
- 5: Input portion
- 6: Output portion
- 21: Travelling member
- 22: Rotating member
- 22a: Holding portion
- 30: Percussion portion
- 31: Hammer portion
- 31a: Elongated member
- 31b: Touching portion
- 32: Driving portion
- 32a: Cam
- 32b: Contact body
- 32c: Revolving portion
- 32d: Biasing member
- 32e: Restriction portion
- 32f: Sliding contact portion
- 32g: Recessed portion
- 32h: Locking piece
- 33: Detection portion
- 41: Calculation portion
- 41a: First impingement calculation portion
- 41b: Second impingement calculation portion
- 41c: Third impingement calculation portion
- 42: Determination portion
- 61: Display portion
- 62: Sorting portion
- 100: Egg (object)

## Claims

1. An examination device (1) comprising: a hammer portion (31) configured to tap an egg (100), said hammer portion (31) including an elongated member (31a) formed to have an elongated length, and a touching portion (31b) which is coupled to a tip end portion of the elongated member (31a) and touches on the egg (100); a driving portion (32) configured to operate the hammer portion (31), said driving portion (32) continuously biasing the hammer portion (31) toward the egg (100) during a single tapping operation of the hammer portion so that the touching portion (31) impinges on the egg (100) multiple times; a piezoelectric detection portion (33) configured to detect a vibration caused by the hammer portion (31) tapping the egg (100); a calculation portion (41) configured to calculate a number of times the hammer portion (31) has impinged on the egg (100) regarding a single tapping operation of the hammer portion (31), based on the vibration detected by the detection portion (33); and a determination portion (42) configured to determine quality of the egg (100), based on the number of times calculated by the calculation portion (41), **characterized in that** the piezoelectric detection portion (33) is mounted to the touching portion (31b) and the elongated member (31a) has elasticity such that the elongated member (31a) warps when the hammer portion (31) taps the egg (100).

2. The examination device (1) according to claim 1, wherein
the driving portion (32) includes a rotatable cam (32a), a rod-shaped contact body (32b) which comes into contact with the cam (32a) at its tip end portion, and a revolving portion (32c) which is coupled to base end portion of the elongated member (31a) and base end portion of the contact body (32b) and causes the hammer portion (31) and the contact body (32b) to revolve about an axis.

3. The examination device (1) according to claim 2, wherein
the driving portion (32) includes a biasing member (32d) for biasing the touching portion (31b) of the hammer portion (31) toward the egg (100).

4. The examination device (1) according to claim 2 or 3, wherein
the driving portion (32) includes a restriction portion (32e) configured to restrict the hammer portion (31) and the contact body (32b) from revolving by a predetermined angle or more since the contact body (32b) touches therewith.

5. The examination device (1) according to any one of claims 2 to 4, wherein
the cam (32a) includes, at its outer peripheral portion, a sliding contact portion (32f) which comes into sliding contact with the contact body (32b), in order to hold the hammer portion (31) at a standby position at which the hammer portion (31) is on standby in such a way as to be spaced apart from the egg (100).

6. The examination device (1) according to claim 5, wherein
the cam (32a) includes a recessed portion (32g) for releasing the contact of the cam (32a) with the contact body (32b) for causing the hammer portion (31) to tap the egg (100).

7. The examination device (1) according to claim 6, wherein
the cam (32a) includes a locking piece (32h) for locking the contact body (32b), in order to restore the hammer portion (31) to the standby position.

8. The examination device (1) according to any one of claims 1 to 7, wherein
the calculation portion (41) is configured to calculate a number of times the vibration strength of the vibration detected by the detection portion (33) has exceeded a set value, as a number of impingements, and
the determination portion (42) is configured to determine that the egg (100) is good when the number of impingements calculated by the calculation portion (41) is equal to or more than a set number of times, and to determine that the egg (100) is not good, when the number of impingements calculated by the calculation portion (41) is less than the set number of times.

9. The examination device (1) according to any one of claims 1 to 7, wherein
the calculation portion (41) includes a first impingement calculation portion (41a) configured to calculate time of a first impingement, and a second impingement calculation portion (41b) configured to determine, through a calculation, presence or absence of a second impingement,
the first impingement calculation portion (41a) calculates a largest vibration strength, as a first impingement, out of the vibration detected by the detection portion (33),
the second impingement calculation portion (41b) detects a largest vibration strength within a set time period after the first impingement, and the second impingement calculation portion (41b) determines that there has been a second impingement when this vibration strength is larger than a set value and determines that there has been no second impingement when this vibration strength is smaller than the set value, and
the determination portion (42) is configured to determine that the egg (100) is not good, when the second impingement calculation portion (41b) determines that there has been no second impingement.

10. The examination device (1) according to claim 9, wherein
the calculation portion (41) further includes a third impingement calculation portion (41c) configured to determine, through a calculation, presence or absence of a third impingement, and
the third impingement calculation portion (41c) does not determine, through a calculation, presence or absence of a third impingement, when the second impingement calculation portion (41b) determines that there has been no second impingement.

11. The examination device (1) according to claim 10, wherein
the third impingement calculation portion (41c) is configured to detect a largest vibration strength within a set time period after the second impingement when the second impingement calculation portion (41b) determines that there has been the second impingement, and the third impingement calculation portion (41c) determines that there has been a third impingement when this vibration strength is larger than a set value and determines that there has been no third impingement when this vibration strength is smaller than the set value, and
the determination portion (42) is configured to determine that the egg (100) is not good, when the third impingement calculation portion (41c) determines that there has been no third impingement.

## Patentansprüche

1. Untersuchungsvorrichtung (1), umfassend:
einen Hammerabschnitt (31), der konfiguriert ist, um ein Ei (100) zu klopfen, wobei der Hammerabschnitt (31) ein verlängertes Element (31) einschließt, das gebildet ist, um eine verlängerte Länge und einen Berührungsabschnitt (31b) aufzuweisen, der mit einem Spitzenendabschnitt des verlängerten Elements (31a) gekoppelt ist und das Ei (100) antastet; einen Antriebsabschnitt (32), der konfiguriert ist, um den Hammerabschnitt (31) anzutreiben, wobei der Antriebsabschnitt (32) während eines einzelnen Klopfvorgangs des Hammerabschnitts den Hammerabschnitt (31) kontinuierlich in Richtung des Eis (100) vorspannt, sodass der Berührungsabschnitt (31) mehrere Male auf dem Ei (100) auftrifft; einen piezoelektrischen Detektionsabschnitt (33), der konfiguriert ist, um eine durch den Hammerabschnitt (31), der das Ei (100) klopft, verursachte Vibration zu detektieren; einen Berechnungsabschnitt (41), der konfiguriert ist, um, basierend auf der durch den Detektionsabschnitt (33) detektierten Vibration, eine Anzahl von Malen zu berechnen, die der Hammerabschnitt (31) im Hinblick auf einen einzelnen Klopfvorgang des Hammerabschnitts (31) auf dem Ei (100) aufgetroffen ist; und einen Bestimmungsabschnitt (42), der konfiguriert ist, um die Qualität des Eis (100), basierend auf der durch den Berechnungsabschnitt (41) berechneten Anzahl von Malen zu bestimmen, **dadurch gekennzeichnet, dass** der piezoelektrische Detektionsabschnitt (33) an dem Berührungsabschnitt (31b) montiert ist und das verlängerte Element (31a) eine Elastizität derart aufweist, dass das verlängerte Element (31a) sich verdreht, wenn der Hammerabschnitt (31) das Ei (100) klopft.

2. Untersuchungsvorrichtung (1) nach Anspruch 1, wobei
der Antriebsabschnitt (32) eine drehbare Nocke (32a), einen stabförmigen Kontaktkörper (32b), der mit der Nocke (32a) an ihrem Spitzenendabschnitt in Kontakt kommt, und einen drehenden Abschnitt (32c) einschließt, der mit dem Basisendabschnitt des verlängerten Elements (31a) und dem Basisendabschnitt des Kontaktkörpers (32b) gekoppelt ist und den Hammerabschnitt (31) und den Kontaktkörper (32b) veranlasst, sich um eine Achse zu drehen.

3. Untersuchungsvorrichtung (1) nach Anspruch 2, wobei
der Antriebsabschnitt (32) ein Vorspannelement (32d) zum Vorspannen des Berührungsabschnitts (31b) des Hammerabschnitts (31) in Richtung des Eis (100) einschließt.

4. Untersuchungsvorrichtung (1) nach Anspruch 2 oder 3, wobei
der Antriebsabschnitt (32) einen Restriktionsabschnitt (32e) einschließt, der konfiguriert ist, um den Hammerabschnitt (31) und den Kontaktkörper (32b) daran zu hindern, sich um einen vorbestimmten Winkel oder mehr zu drehen, wenn der Kontaktkörper (32b) damit in Berührung kommt.

5. Untersuchungsvorrichtung (1) nach einem der Ansprüche 2 bis 4, wobei
die Nocke (32a) an ihrem Außenumfangsabschnitt einen gleitenden Kontaktabschnitt (32f) einschließt, der mit dem Kontaktkörper (32b) in gleitenden Kontakt kommt, um den Hammerabschnitt (31) in einer Bereitschaftsposition zu halten, in welcher der Hammerabschnitt (31) auf eine solche Weise in Bereitschaft ist, um von dem Ei (100) beabstandet zu sein.

6. Untersuchungsvorrichtung (1) nach Anspruch 5, wobei
die Nocke (32a) einen Aussparungsabschnitt (32g) einschließt, um den Kontakt der Nocke (32a) mit dem Kontaktkörper (32b) freizugeben, um den Hammerabschnitt (31) zu veranlassen, das Ei (100) zu klopfen.

7. Untersuchungsvorrichtung (1) nach Anspruch 6, wobei
die Nocke (32a) ein Verriegelungsteil (32h) zum Verriegeln des Kontaktkörpers (32b) einschließt, um den Hammerabschnitt (31) in die Bereitschaftsposition zurückzuführen.

8. Untersuchungsvorrichtung (1) nach einem der Ansprüche 1 bis 7, wobei
der Berechnungsabschnitt (41) konfiguriert ist, um eine Anzahl von Malen, welche die Vibrationsstärke der durch den Detektionsabschnitt (33) detektierten Vibration einen eingestellten Wert überschritten hat, als eine Anzahl von Auftreffen zu berechnen, und
der Bestimmungsabschnitt (42) konfiguriert ist, um zu bestimmen, dass das Ei (100) gut ist, wenn die Anzahl von durch den Berechnungsabschnitt (41) berechneten Auftreffen gleich oder höher als eine eingestellte Anzahl von Malen ist, und zu bestimmen, dass das Ei (100) nicht gut ist, wenn die Anzahl von durch den Berechnungsabschnitt (41) berechneten Auftreffen niedriger als die eingestellte Anzahl von Malen ist.

9. Untersuchungsvorrichtung (1) nach einem der Ansprüche 1 bis 7, wobei
der Berechnungsabschnitt (41) einen ersten Auftreffberechnungsabschnitt (41a), der konfiguriert ist, um den Zeitpunkt eines ersten Auftreffens zu berechnen, und einen zweiten Auftreffberechnungsabschnitt (41b) einschließt, der konfiguriert ist, um durch eine Berechnung die Anwesenheit oder Abwesenheit eines zweiten Auftreffens zu bestimmen,
der erste Auftreffberechnungsabschnitt (41a) aus den durch den Detektionsabschnitt (33) detektierten Vibrationen eine größte Vibrationsstärke als ein erstes Auftreffen berechnet,
der zweite Auftreffberechnungsabschnitt (41b) eine größte Vibrationsstärke innerhalb eines eingestellten Zeitraums nach dem ersten Auftreffen detektiert, und der zweite Auftreffberechnungsabschnitt (41b) bestimmt, dass es ein zweites Auftreffen gab, wenn diese Vibrationsstärke größer als ein eingestellter Wert ist und bestimmt, dass es kein zweites Auftreffen gab, wenn die Vibrationsstärke kleiner als der eingestellte Wert ist, und
der Bestimmungsabschnitt (42) konfiguriert ist, um zu bestimmen, dass das Ei (100) nicht gut ist, wenn der zweite Auftreffberechnungsabschnitt (41b) bestimmt, dass es kein zweites Auftreffen gab.

10. Untersuchungsvorrichtung (1) nach Anspruch 9, wobei der Berechnungsabschnitt (41) weiter einen dritten Auftreffberechnungsabschnitt (41c) einschließt, der konfiguriert ist, um durch eine Berechnung die Anwesenheit oder Abwesenheit eines dritten Auftreffens zu bestimmen, und
der dritte Auftreffberechnungsabschnitt (41c) nicht durch eine Berechnung die Anwesenheit oder Abwesenheit eines dritten Auftreffens bestimmt, wenn der zweite Auftreffberechnungsabschnitt (41b) bestimmt, dass es kein zweites Auftreffen gab.

11. Untersuchungsvorrichtung (1) nach Anspruch 10, wobei der dritte Auftreffberechnungsabschnitt (41c), konfiguriert, um eine größte Vibrationsstärke innerhalb eines eingestellten Zeitraums nach dem zweiten Auftreffen zu detektieren, wenn der zweite Auftreffberechnungsabschnitt (41b) bestimmt, dass es das zweite Auftreffen gab, und der dritte Auftreffberechnungsabschnitt (41c) bestimmt, dass es ein drittes Auftreffen gab, wenn diese Vibrationsstärke größer als ein eingestellter Wert ist und bestimmt, dass es kein drittes Auftreffen gab, wenn diese Vibrationsstärke kleiner als der eingestellte Wert ist, und
der Bestimmungsabschnitt (42) konfiguriert ist, um zu bestimmen, dass das Ei (100) nicht gut ist, wenn der Auftreffberechnungsabschnitt (41c) bestimmt, dass es kein drittes Auftreffen gab.

## Revendications

1. Dispositif d'examen (1) comprenant :
une unité de marteau (31) configurée pour frapper un œuf (100), ladite unité de marteau (31) incluant un élément allongé (31a) formé pour présenter une longueur allongée, et une portion jointive (31b) qui est couplée à une partie d'extrémité de pointe de l'élément allongé (31a) et qui touche l'œuf (100) ; une unité d'entraînement (32) configurée pour faire fonctionner l'unité de marteau (31), ladite unité d'entraînement (32) sollicite continuellement l'unité de marteau (31) vers l'œuf (100) lors d'une seule action de frappe de l'unité de marteau de sorte que la portion jointive (31) cogne sur l'œuf (100) plusieurs fois; une unité de détection piézoélectrique (33) configurée pour détecter une vibration causée par l'unité de marteau (31) frappant sur l'œuf (100) ; une unité de calcul (41) configurée pour calculer le nombre de fois que l'unité de marteau (31) a cogné l'œuf (100) en relation avec une seule action de frappe de l'unité de marteau (31), sur la base des vibrations détectées par l'unité de détection (33) ; et une unité d'évaluation (42) configurée pour déterminer la qualité de l'œuf (100), sur la base du nombre de fois calculée par l'unité de calcul (41) **caractérisée en ce que** l'unité de détection piézoélectrique (33) est montée sur la portion jointive (31b) et l'élément allongé (31a) présente une élasticité telle que l'élément allongé (31a) se déforme quand l'unité de marteau (31) frappe l'œuf (100).

2. Dispositif d'examen (1) selon la revendication 1 dans lequel l'unité d'entraînement (32) inclut une came rotative (32a), un corps de contact (32b) en forme de tige qui entre en contact avec la came (32a) à sa partie d'extrémité en pointe, et une unité rotative (32c) qui est couplée à la partie d'extrémité de la base de l'élément allongé (31a) et à la partie d'extrémité de la base du corps de contact (32b) et amène l'unité de marteau (31) et le corps de contact (32b) à tourner autour d'un axe.

3. Dispositif d'examen (1) selon la revendication 2, dans lequel l'unité d'entraînement (32) inclut un élément de sollicitation (32d) pour solliciter la portion jointive (31b) de l'unité de marteau (31) vers l'œuf (100).

4. Dispositif d'examen (1) selon la revendication 2 ou 3, dans lequel
l'unité d'entraînement (32) inclut une portion de contention (32e) configurée pour empêcher l'unité de marteau (31) et le corps de contact (32b) de tourner d'un angle prédéterminé ou plus puisque le corps de contact (32b) est en contact avec ceux-ci.

5. Dispositif d'examen (1) selon l'une quelconque des revendications 2 à 4, dans
lequel
la came (32a) inclut, à sa partie périphérique externe, une partie en contact coulissant (32f) qui entre en contact coulissant avec le corps de contact (32b), de sorte à contenir l'unité de marteau (31) dans une position d'attente dans laquelle l'unité de marteau (31) est en attente de manière à être espacée de l'œuf (100).

6. Dispositif d'examen (1) selon la revendication 5, dans lequel
la came (32a) inclut une portion renfoncée (32g) pour libérer le contact entre la came (32a) et le corps de contact (32b) pour amener l'unité de marteau (31) à frapper l'œuf (100).

7. Dispositif d'examen (1) selon la revendication 6, dans lequel
la came (32a) inclut une pièce de blocage (32h) pour bloquer le corps de contact (32b) afin de restaurer l'unité de marteau (31) dans la position d'attente.

8. Dispositif d'examen (1) selon l'une quelconque des revendications 1 à 7, dans
lequel
l'unité de calcul (41) est configurée pour calculer un nombre de fois où la force de vibration de la vibration détectée par l'unité de détection (33) a dépassé une valeur déterminée, comme un nombre d'impacts, et
l'unité d'évaluation (42) est configurée pour déterminer que l'œuf (100) est bon lorsque le nombre d'impacts calculé par l'unité de calcul (41) est égal ou supérieur à un nombre de fois déterminé, et pour déterminer que l'œuf (100) n'est pas bon, lorsque le nombre d'impacts calculé par l'unité de calcul (41) est inférieur au nombre de fois déterminé.

9. Dispositif d'examen (1) selon l'une quelconque des revendications 1 à 7, dans
lequel
l'unité de calcul (41) inclut une unité de calcul de premier impact (41a) configurée pour calculer le temps d'un premier impact, et une unité de calcul de deuxième impact (41b) configurée pour déterminer, par un calcul, la présence ou l'absence d'un deuxième impact,
l'unité de calcul de premier impact (41a) calcule une force de vibration la plus large, en tant que premier impact, à partir de la vibration détectée par l'unité de détection (33)
l'unité de calcul de deuxième impact (41b) détecte une force de vibration la plus large dans une période de temps déterminée après le premier impact, et l'unité de calcul de deuxième impact (41b) détermine qu'il y a eu un deuxième impact lorsque cette force de vibration est plus grande qu'une valeur déterminée et détermine qu'il n'y a pas eu de deuxième impact lorsque cette force de vibration est plus petite que la valeur déterminée, et
l'unité d'évaluation (42) est configurée pour déterminer que l'œuf (100) n'est pas bon, lorsque l'unité de calcul de deuxième impact (41b) détermine qu'il n'y a pas eu de deuxième impact.

10. Dispositif d'examen (1) selon la revendication 9, dans lequel
l'unité de calcul (41) inclut en outre une unité de calcul de troisième impact (41c) configurée pour déterminer, par un calcul, la présence ou l'absence d'un troisième impact, et
l'unité de calcul de troisième impact (41c) ne détermine pas, par un calcul, la présence ou l'absence d'un troisième impact, lorsque l'unité de calcul de deuxième impact (41b) détermine qu'il n'y a pas eu de deuxième impact.

11. Dispositif d'examen (1) selon la revendication 10, dans lequel
l'unité de calcul de troisième impact (41c) est configurée pour détecter une force de vibration la plus large lorsque l'unité de calcul de deuxième impact (41b) détermine qu'il y a eu le deuxième impact, et l'unité de calcul de troisième impact (41c) détermine qu'il y a eu un troisième impact lorsque cette force de vibration est plus grande qu'une valeur déterminée et détermine qu'il n'y a pas eu de troisième impact lorsque cette force de vibration est plus petite que la valeur déterminée, et
l'unité d'évaluation (42) est configurée pour déterminer que l'œuf (100) n'est pas bon, lorsque l'unité de calcul de troisième impact (41c) détermine qu'il n'y a pas eu de troisième impact.
